# EUROPEAN PATENT SPECIFICATION

(11) **EP 0 997 453 B1**
(45) Date of publication and mention of the grant of the patent: **05.02.2003**
(21) Application number: 99120387.8
(22) Date of filing: 13.10.1999
(51) Int. Cl.: C07C 59/68, C07C 229/12, C07C 229/22, C07C 215/40, A61K 31/205, A61P 1/16

(54) **Phenoxyisobutyric acid ammonium salts and pharmaceutical formulations containing them**
Ammoniumsalze von Phenoxy-Isobuttersäure und diese enthaltende pharmazeutische Formulierungen
Sels d'ammonium de l'acide phénoxy-isobutyrique et formulations pharmaceutiques les renfermant

(30) Priority: 27.10.1998 IT MI982300
(43) Date of publication of application: 03.05.2000
(73) Proprietor: FATRO S.p.A., I-40064 Ozzano Emilia (Bologna) (IT)
(72) Inventor: Melloni, William, 40064 Ozzano Emilia (BO) (IT); Zaini, Corrado, 40064 Ozzano Emilia (BO) (IT)
(74) Representative: Minoja, Fabrizio, Dr.

(56) References cited:
- FR-M- 2 215
- GB-A- 1 494 299
- CHEMICAL ABSTRACTS, vol. 78, no. 9, 5 March 1973 (1973-03-05) Columbus, Ohio, US; abstract no. 58070z, page 489; XP002130130 & RO 52 775 A (INSTITUTUL DE CERCETARI CHIMICO-FARMACEUTICE)
- DATABASE REGISTRY FILE [Online] Chemical Abstract Service XP002130131 & FIZIOL. AKT. VESHCHESTVA, vol. 9, 1977, pages 56-9,

## Description

The present invention relates to phenoxyisobutyric acid quaternary ammonium salts, the processes for the preparation thereof and pharmaceutical formulations for the prevention and therapy of diseases in human and veterinary medicine.

Phenoxyisobutyric acid (or 2-methyl-2-phenoxypropionic acid) is used as sodium salt in veterinary medicine (HEPAGEN^{(R)}) both as choleretic and hepatoprotecting drug.

Phenoxyisobutiric acid and its sodium salt have been also disclosed in Chem. Abs. 78 (1973) Abs.n° 58070 Z as having antihypercholesteremic activity. Fiziol. Akt. Veshchestva (1977), 9, 56-9 discloses a salt of phenoxyisobutiric acid with 2-aminoethanol as anticholesteremic agent.

Phenoxyisobutyric acid derivatives salts with amino acid pyridinyl methyl esters for the treatment of hyperlipidemias are disclosed in US 4,000,284.

2-(p-Chlorophenoxy)-2-methyl propionic acid salt with amino acids for the treatment of hyperlipidemias are described in GB 1,494,299.

Phenoxyisobutyric acid, being water insoluble, is not used as it is but in the form of alkali metal salt, preferably as sodium salt. The problem with phenoxyisobutyric acid sodium salt is, however, its remarkable irritant action at the injection site as well as poor bioavailability.

It has now surprisingly been found, and this is object of the present invention, that phenoxyisobutyric acid quaternary ammonium salts have improved tolerability, bioavailability and effectiveness compared with the sodium salt.

A further advantage of the present invention is that phenoxyisobutyric acid quaternary ammonium salts, such as Betaine, Choline and dl- or l-Carnitine salts, improve or enhance the detoxifying, hepatoprotecting and choleretic actions of the free acid.

The salts of the invention are readily soluble in water at pH of 7 - 7.5 and can be used in pharmaceutical formulations for the oral, rectal, topical and parenteral administrations, such as tablets, syrups, capsules, suppositories, pessaries, ointments, solutions or lyophylized forms for the extemporary use, in admixture with suitable excipients, carriers or solvents conventionally used in the pharmaceutical field.

Said pharmaceutical formulations containing phenoxyisobutyric acid quaternary ammonium salts are a further object of the invention.

Pharmaceutical formulations for the oral or parenteral use can be obtained by simple combination of the phenoxyisobutyric acid sodium salt in solution with an equivalent amount or an excess of the quaternary ammonium salifying agent.

In these formulations, which are a further object of the invention, the molar ratio of phenoxyisobutyric acid salt to salifying quaternary agent can range from 10:1 to 1:10.

### Detailed disclosure of the invention

The phenoxyisobutyric acid quaternary ammonium salts are represented by formula I: 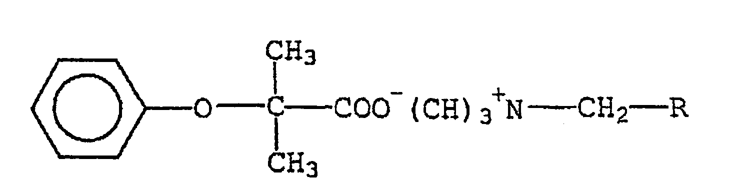 wherein R is C₁-C₃ alkyl containing hydroxy and/or carboxy groups.

R is preferably a group of formula -CH₂-OH, -CHOH-CH₂-COOX, -COOX and X is H, Na, K or alkaline-earth metals cations.

Preferred ammonium cations are Betaine, Choline and 1-or dl-Carnitine.

The compounds of the invention are prepared by reacting phenoxyisobutyric acid or its sodium salt with the quaternary ammonium compound, in the form of inner salt, hydrochloride or hydroxide, in a suitable solvent.

Phenoxyisobutyric acid is preferably reacted with the ammonium hydroxide salt in a suitable solvent.

Organic solvents, such as acetone, isopropanol, ethanol, methanol and the mixtures thereof with water can be used as solvents.

The following examples further disclose the invention.

### Example 1

Betaine phenoxyisobutyrate sodium salt.

3.9 g (0.022 mols) of phenoxyisobutyric acid are dissolved in 10 ml of acetone.

Separately, a solution of 2.9 g (0.022 mols) of Betaine monohydrate in 2 ml of water is added with 2.2 ml (0.022 mols) of a 10N NaOH solution.

The Betaine basic solution is slowly poured into the phenoxyisobutyric acid solution. The resulting solution is concentrated to about 4 ml and slowly added to 100 ml of acetone.

A white solid precipitates, that is stirred for about 20 minutes, then filtered and washed with some acetone. The resulting solid is recovered and dried under vacuum. 5.2 g of the title compound are obtained, yield = 74%.
C₁₀H₁₁O₃·C₅H₁₁NO₂·Na M.W. = 319.26
M.p. 83 - 84°C
¹H-NMR (DMSO-d₆): δ 1.4 (6H, s) - 2.5 (2H, m), 3.15 - 3.5 (9H, m), 6.8 (3H, m) - 7.1 (2H, m).

### Example 2

l-Carnitine phenoxyisobutyrate.

1.8 g (0.01 mols) of phenoxyisobutyric acid are dissolved in 8 ml of acetone.

Separately, a solution of 1.6 g (0.01 mols) of 1-Carnitine in 3 ml of H₂O is added with 1 ml (0.01 mols) of 10N NaOH.

The l-Carnitine solution is slowly poured into the phenoxyisobutyric acid solution. After stirring for about 5 minutes, the mixture is evaporated to nearly dryness under vacuum (rotatory evaporator) on 40°C bath.

The residue is taken up with about 80 ml of acetone and stirred for 20 minutes. The resulting white solid is filtered and dried under vacuum at 30°C. 2.2 g of the title compound are obtained, yield = 60%.
C₁₀H₁₁O₃·C₇H₁₅NO₃·Na
Highly hygroscopic solid
M.p. = 145° (Decomp.)
¹H-NMR (DMSO-d₆/D₂O): δ 1.35 (6H, s) - 2.1 (2H, m) , 3.05 (9H, s), 3.25 (2H, m) - 3.8 (s, H₂O), 4.25 (1H, d), 6.75 (3H, m), 7.15 (2H, m) .

### Example 3

Choline phenoxyisobutyrate.

A mixture of 2.20 g (0.01 mols) of sodium phenoxyisobutyrate in 50 ml of isopropyl alcohol is slowly added with 1.39 g (0.01 mols) of choline chloride.

The mixture is refluxed for about 1 hour, then cooled at room temperature under stirring. Sodium chloride is filtered off and the filtrate is evaporated under vacuum. The solid is taken up with about 10 ml of acetone, to obtain a white solid. 1.3 g of the title compound are obtained, yield = 46%.
C₁₀H₁₁O₃·C₅H₁₄NO - M.W. = 283.38
M.p. = 34.6°C
H¹-NMR (DMSO-d₆): δ 1.4 (6H, s) - 3.1 (9H, s), 3.4 (2H, m), 3.8 (2H, s) - 6.75 (3H, m), 7.15 (2H, m).

## Claims

1. Compounds of formula: 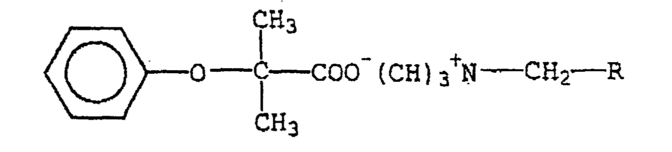 wherein R is a C₁-C₃ alkyl group containing hydroxy and/or carboxy groups.

2. A compound according to claim 1, wherein R is CH₂-OH, -CHOH-CH₂-COOX, -COOX, with X = H, Na, K or alkaline-earth metals.

3. A compound as claimed in claim 1 or 2, in which R is CH₂OH (Choline phenoxyisobutyrate).

4. A compound as claimed in claim 1 or 2, wherein R is COONa (Betaine phenoxyisobutyrate sodium salt).

5. A compound as claimed in claim 1 or 2, wherein R is CHOH-CH₂-COONa (sodium dl- or l-Carnitine phenoxyisobutyrate).

6. A process for the production of the compounds as claimed in claim 1, which comprises reacting phenoxyisobutyric acid or an alkali salt thereof with the ammonium salt, in the form of inner or basic salt, in a suitable solvent.

7. Pharmaceutical composition comprising at least one a compound of claim 1 as active ingredient, together with pharmaceutically acceptable excipients.

## Patentansprüche

1. Verbindungen der Formel: 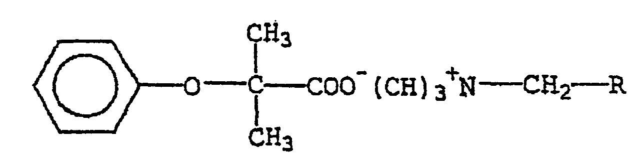 worin R eine C₁-C₃-Alkylgruppe ist, die Hydroxy- und/oder Carboxygruppen enthält.

2. Verbindung nach Anspruch 1, wobei R CH₂-OH, -CHOH-CH₂-COOX, -COOX, mit X = H, Na, K oder Erdalkalimetall ist.

3. Verbindung nach Anspruch 1 oder 2, wobei R CH₂OH ist (Cholinphenoxyisobutyrat).

4. Verbindung nach Anspruch 1 oder 2, worin R COONa ist (Betainphenoxyisobutyrat Natriumsalz).

5. Verbindung nach Anspruch 1 oder 2, worin R CHOH-CH₂-COONa ist (Natrium dl- oder 1-Carnitinphenoxyisobutyrat).

6. Verfahren zur Herstellung der Verbindungen nach Anspruch 1, umfassend die Umsetzung von Phenoxyisobuttersäure oder einem Alkalisalz davon mit dem Ammoniumsalz in Form eines inneren oder basischen Salzes in einem geeigneten Lösungsmittel.

7. Pharmazeutische Zusammensetzung, umfassend mindestens eine Verbindung nach Anspruch 1 als aktiven Bestandteil zusammen mit pharmazeutisch annehmbaren Arzneimittelträgern.

## Revendications

1. Composés de formule : 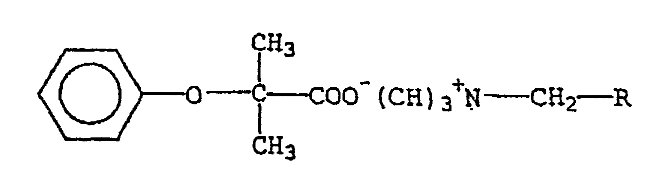 dans laquelle R est un groupe alkyle en C₁-C₃ contenant des groupes hydroxy et/ou carboxy.

2. Composé selon la revendication 1, pour lequel R est CH₂-OH,
-CHOH-CH₂-COOX, -COOX, avec X = H, Na, K ou métaux alcalino-terreux.

3. Composé tel que revendiqué dans la revendication 1 ou 2, pour lequel R est CH₂OH (phénoxyisobutyrate de choline).

4. Composé tel que revendiqué dans la revendication 1 ou 2, pour lequel R est COONa (sel de sodium du phénoxyisobutyrate de bétaïne).

5. Composé tel que revendiqué dans la revendication 1 ou 2, où R est CHOH-CH₂-COONa (phénoxyisobutyrate de dl- ou l-carnitine sodique).

6. Procédé pour la production des composés tels que revendiqués dans la revendication 1, qui comprend la réaction de l'acide phénoxyisobutyrique ou d'un sel alcalin de celui-ci avec le sel d'ammonium, sous la forme d'un sel interne ou basique, dans un solvant approprié.

7. Composition pharmaceutique comprenant au moins un composé de la revendication 1, en tant qu'ingrédient actif, conjointement avec des excipients pharmaceutiquement acceptables.
